# EUROPÄISCHE PATENTSCHRIFT

(11) **EP 1 714 633 B1**
(45) Veröffentlichungstag und Bekanntmachung des Hinweises auf die Patenterteilung: **04.03.2009**
(21) Anmeldenummer: 06007348.3
(22) Anmeldetag: 07.04.2006
(51) Int. Cl.: A61K 6/083

(54) **Dentalmaterialien, enthaltend dendritische Verbindungen**
Dental materials comprising dendritic compounds
Matériaux dentaires contenant les composés dendritiques

(30) Priorität: 20.04.2005 DE 102005018451
(43) Veröffentlichungstag der Anmeldung: 25.10.2006
(73) Patentinhaber: Heraeus Kulzer GmbH, 63450 Hanau (DE)
(72) Erfinder: Ruppert, Klaus, Dr., 63477 Maintal (DE); Grundler, Andreas, Dr., 42117 Wuppertal (DE); Reischl, Kurt, 35799 Merenberg (DE)
(74) Vertreter: Kühn, Hans-Christian

(56) Entgegenhaltungen:
- WO-A-03/013379
- WO-A-20/05014679

## Beschreibung

Die Erfindung betrifft Dentalmaterialien, enthaltend dendritische Verbindungen.

Dendrimere bzw. hyperverzweigte Polymere sind oligo- bzw. polymere Verbindungen, welche sich um ein Zentralmolekül schalenartig zu einem hochverzweigten, mehr oder weniger symmetrischen Grundgerüst aufbauen, und die an der äußersten Hülle mit funktionellen Gruppen versehen sein können. Sie.werden im folgenden als dendritische Verbindungen bezeichnet.

Die Unterscheidung zwischen Dendrimeren und hyperverzweigten Polymeren erfolgt über die Molekülsymmetrie: Während Dendrimere durch einen strenge Reaktionsführung hochsymmetrisch aufgebaut sind, zeigen hyperverzweigte Polymere aufgrund statistischer Verzweigung teilweise Asymmetrien, sind allerdings deutlich kostengünstiger herzustellen.

Dendrimere bzw. hyperverzweigte Polymere werden insbesondere zur Verbesserung der mechanischen Eigenschaften von Polymeren eingesetzt. Dendrimere in füllstoffhaltigen, feinkörnigen, stopfbaren, also amalgamähnlichen Dentalmaterialien wurden in DE 44 43 702 A1 beschrieben. US 200300114553 A1 betrifft ähnliches, härtbares und selbsttragendes Dentalmaterial, das kristalline Komponenten mit dendrimeren oder hyperverzweigten Strukturen enthalten kann. Der Einsatz von Dendrimeren in rheopexem Dentalmaterial wurde z.B. in US 20020068771A1 beschrieben.

WO2005/014679 offenbart Urethan-Acrylat Dendrimeren mit hyperverzweigten Polyester-Kernen als Dentalkompositen.

Erfindungsgemäß werden bestimmte dendritische Verbindungen, wie in den Ansprüchen 1 - 4 beschrieben, Dentalmaterialien zugesetzt, und es ergeben sich Dentalmaterialien gemäß den Ansprüchen 1 - 4 mit überraschend günstigen Eigenschaften.

Die Erfindung betrifft demgemäß Dentalmaterialien enthaltend mindestens eine dendritische Verbindung aus Kern und zwei Schalen,
➢ deren Kern und erste und zweite Schale über Polyurethan-Gruppen miteinander verknüpft sind,
➢ und deren zweite Schale durch Reaktion mit einem (Meth)acrylat modifiziert ist.

Bevorzugt wird das Polyurethan-Gerüst (Kern, erste und zweite Schale) durch die bekannte Reaktion von Diisocyanaten mit drei- oder mehrwertigen Alkoholen gebildet. Die Diisocyanate haben dabei vorzugsweise 4 - 12 C-Atome, besonders 6 - 10. Ganz besonders bevorzugt ist Hexamethylendiisocyanat. Als Alkohole kommen z.B. Pentaerythritol, Dipentaerithritol oder bevorzugt Trimethylolpropan in Frage.

Zur Modifikation der äußeren Schale geeignete (Meth)acrylate sind in erster Linie Hydroxygruppen tragende Methacrylate und Acrylate, vor allem Hydroxyethylmethacrylat.

Als besonders geeignet haben sich Verbindungen erwiesen, die durch die Formel I wiedergegeben werden:

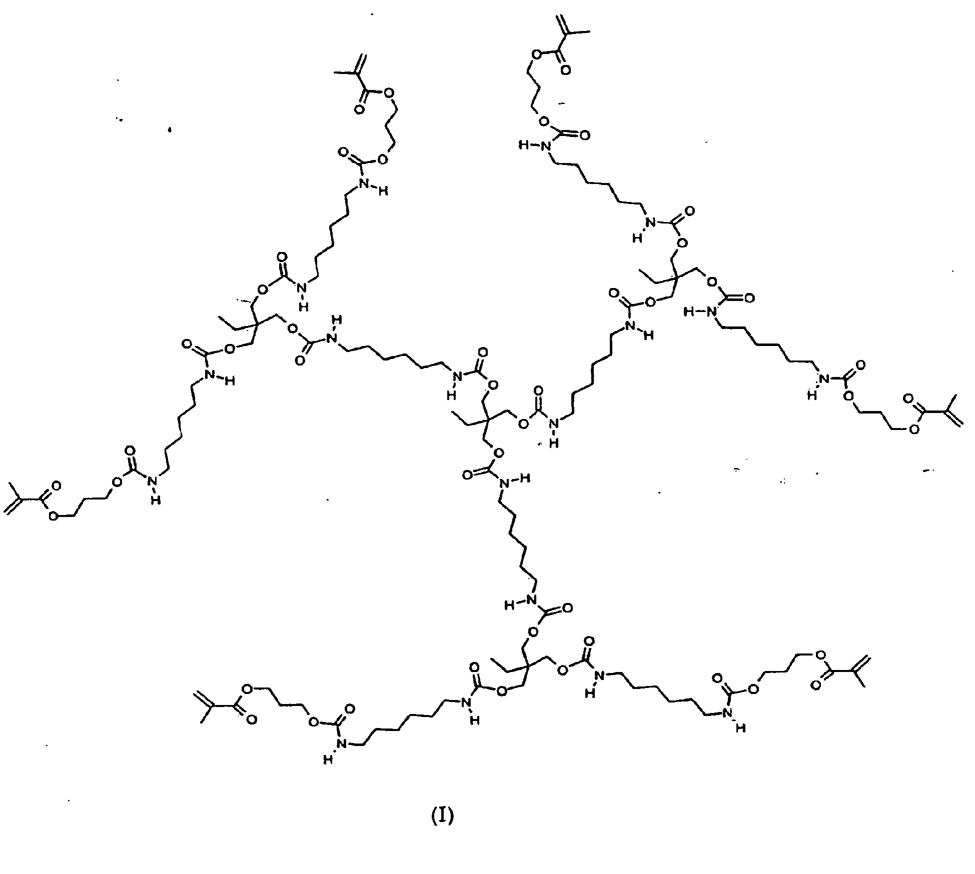

Kennzeichen der Verbindung I sind:
➢ Sie weist zwei Schalen und einen Kern auf.
➢ Die äußere, zweite Schale ist 6-fach HEMA¹-modifiziert.
➢ Der Kern und die Schalen sind über Polyurethan-Gruppen (erhältlich aus Hexamethylendiisocyanat und Trimethylolpropan) verknüpft.
¹ Hydroxyethylmethacrylat

Da Verbindung I das Produkt einer Oligomerisation ist, entspricht dieses Produkt nicht immer in vollkommener Weise der obigen Idealformel. Das Produkt wird in der Regel auch etwas anders geartete, weniger symmetrische Verbindungen enthalten, die als hyperverzweigte Oligomere ebenfalls dendritische Verbindungen sind. Im Vorliegenden sind vom Begriff "Verbindung I" und der Formel I auch derartige, weniger symmetrische Verbindungen umfasst.

Die dendritischen Verbindungen, wie z.B. die Verbindung der Formel I, können nach an sich bekannten Verfahren, wie z.B. analog den in J. Serb. Chem. Soc. 69, 441-453 (2004) beschriebenen, hergestellt werden.

In Frage kommende Dentalmaterialien sind an sich bekannt und z.B. in Ullmann's Encyclopedia of Industrial Chemistry, (CD-Rom 2002), Robert G. Craig, Dieter Welker, Dental Materials, Stand 15. Juni 2000, besonders unter "Cements" und "Synthetic Resins" beschrieben. Bevorzugt sind Kompositmaterialien, besonders Füllungskomposite.

Insbesondere werden die oben beschriebenen dendritischen Verbindungen zur Optimierung der mechanischen Eigenschaften, besonders als Elastifizierer und/oder Rheologieverbesserer, verwendet. Die Biegefestigkeit wird z.B. durch die unten beschriebene Verbindung I um mehr als 10 MPa angehoben, verglichen mit dem gleichen Dentalkomposit ohne Zusatz der Verbindung I. Außerdem kann der unerwünschte Polymerisationsschrumpf günstig beeinflusst werden.

Die erfindungsgemäß eingesetzten dendritischen Verbindungen verbessern auch die rheologischen Eigenschaften der Dentalmaterialien vor der Härtung: Unter Scherbeanspruchung, d.h. beim Modellieren, nimmt die Viskosität der Paste ab. Sobald die Scherung wegfällt, wird ein signifikanter Viskositätsanstieg beobachtet (analog den Aerosilen, aber ohne deren negative Beeinflussung der Produkteigenschaften: Von Aerosilen wird nämlich die Transparenz beeinträchtigt, und ein Aufschluß der Aerosile ist notwendig).

Vorteilhaft ist ein Konzentrationsbereich der dendritischen Verbindungen im Dentalkomposit von
1 - 10 Gew. %, vorzugsweise 1 - 5 Gew. %, ganz besonders bevorzugt 1 - 3 Gew. %.

### Beispiel:

Eine Monomer-Dispersion, bestehend aus den Komponenten
- Bis-GMA 14 Gew.%
- TEDMA 6 Gew.%
- Nicht-agglomerierte SiO₂-Teilchen (20 nm) 9 Gew. %
wird mit 0,01 Gew.% Stabilisator BHT (4-Methyl-2,6-di-tert-butyl-phenol, CAS 128-37-0) sowie den Initiatoren Campherchinon (0,3 Gew.%) und 0,2 Gew.% 2-Ethylhexyl-4-dimethylaminobenzoat (Quantacure™EHA, CAS 21245-02-3, Great Lakes Chemicals) versetzt.

1 Gew. % der Verbindung I werden unter leichtem Erwärmen auf 40 °C zugegeben. Anschließend gibt man ein silanisiertes Dentalglas (Ba-Al-Silikatglas; 70,0 Gew. %) zu. Nach inniger Vermischung ergibt sich ein als Füllungskomposit geeignetes Dentalmaterial.

## Patentansprüche

1. Dentalmaterial enthaltend mindestens eine dendritische Verbindung aus Kern und zwei Schalen,
➢ deren Kern und erste und zweite Schale über Polyurethan-Gruppen miteinander verknüpft sind
➢ und deren zweite Schale durch Reaktion mit einem (Meth)acrylat modifiziert ist.

2. Dentalmaterial gemäß Anspruch 1, wobei die erste und die zweite Schale aus einem
➢ Polyurethan-Gerüst, erhältlich aus der Reaktion von Hexamethylendiisocyanat und Trimethylolpropan, bestehen.

3. Dentalmaterial gemäß Anspruch 1, wobei die
➢ zweite Schale durch Reaktion mit Hydroxyethylmethacrylat modifiziert ist.

4. Dentalmaterial gemäß einem der vorstehenden Ansprüche, wobei die dendritische Verbindung im wesentlichen der Formel I entspricht.

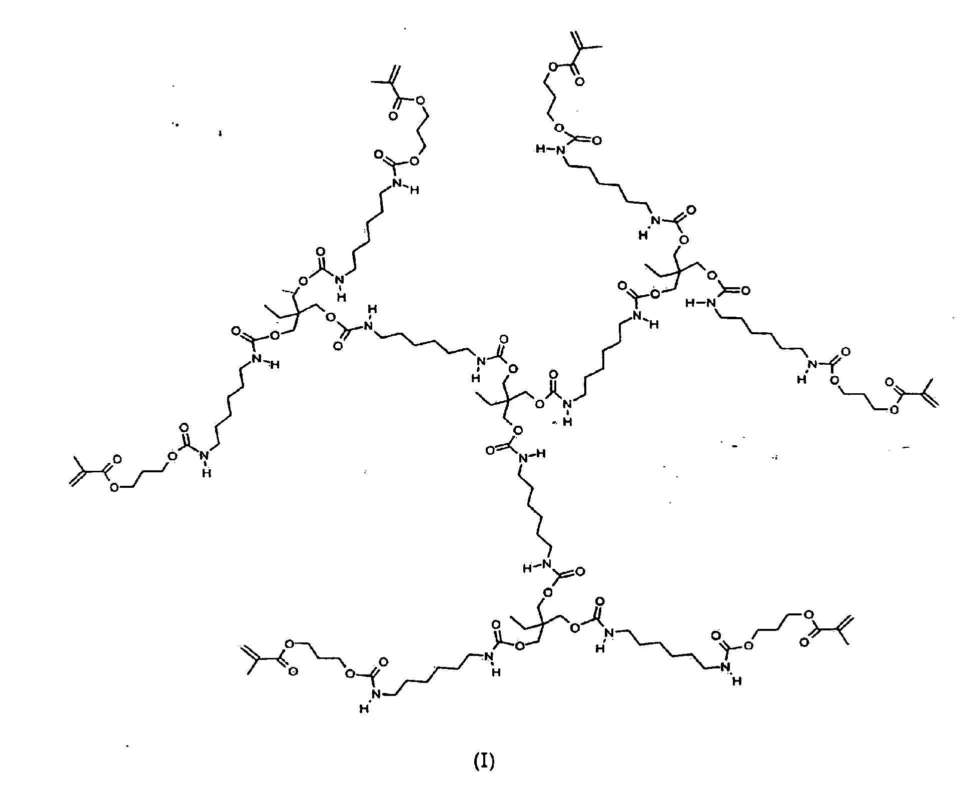

5. Verwendung einer dendritischen Verbindung, wie in einem der vorstehenden Ansprüche 1 bis 4 beschrieben, als Elastifizierer in Dentalmaterialien.

6. Verwendung einer dendritischen Verbindung, wie in einem der vorstehenden Ansprüche 1 bis 4 beschrieben, zur Verbesserung der rheologischen Eigenschaften von Dentalmaterialien.

## Claims

1. Dental material containing at least one dendritic compound consisting of core and two shells, ➢ whose core and first and second shell are linked to each other via polyurethane groups ➢ and whose second shell is modified by reaction with a (meth)acrylate.

2. Dental material according to claim 1, wherein the first and the second shell consist of a
➢ polyurethane framework that can be obtained from the reaction of hexamethylene diisocyanate and trimethylolpropane.

3. Dental material according to claim 1, wherein the
➢ second shell is modified by reaction with hydroxyethylmethacrylate.

4. Dental material according to any one of the preceding claims, wherein the dendritic compounds essentially corresponds to Formula I.

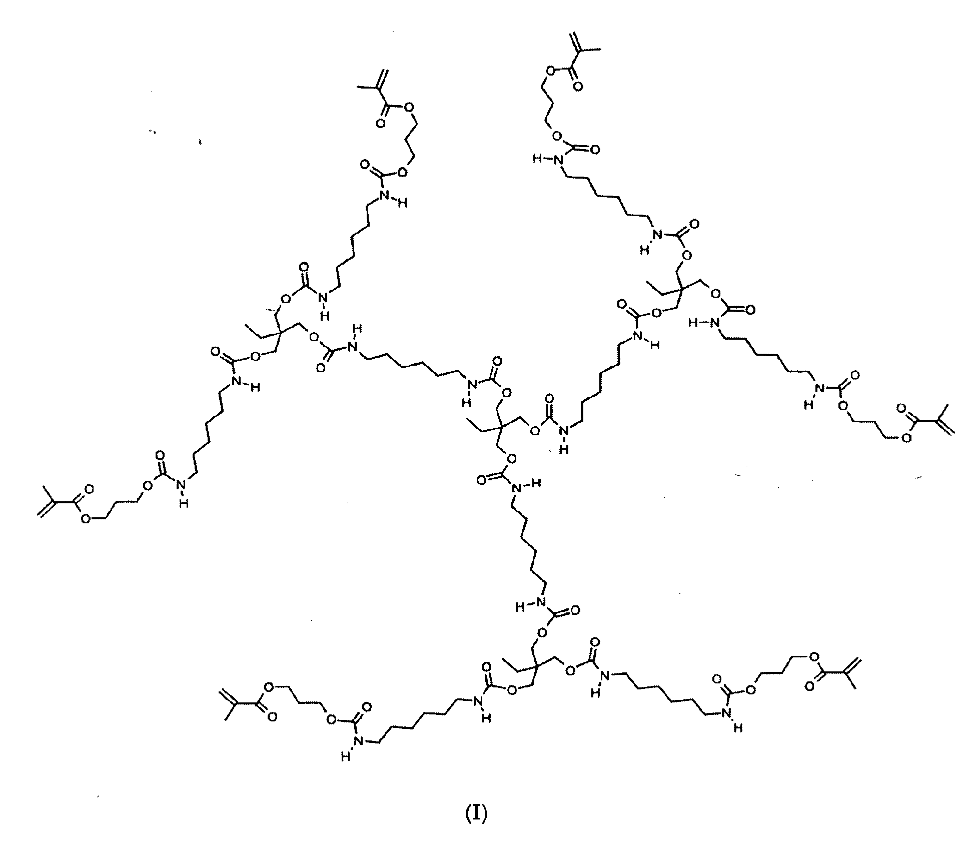

5. Use of a dendritic compound of the type described in any one of the preceding claims 1 to 4, as elasticity-enhancing agent in dental materials.

6. Use of a dendritic compound of the type described in any one of the preceding claims 1 to 4, for improving the rheological properties of dental materials.

## Revendications

1. Matériau dentaire contenant au moins un composé dendritique constitué d'un noyau et de deux enveloppes,
dont le noyau et les première et seconde enveloppes sont reliées ensemble par l'intermédiaire de groupes polyuréthane,
et dont la seconde enveloppe est modifiée par réaction avec un (méth)acrylate.

2. Matériau dentaire selon la revendication 1, dans lequel la première et la seconde enveloppes sont constituées d'une structure de polyuréthane obtenue par la réaction de diisocyanate d'hexaméthylène et de triméthylolpropane.

3. Matériau dentaire selon la revendication 1, dans lequel
la seconde enveloppe est modifiée par réaction avec du méthacrylate d'hydroxyéthyle.

4. Matériau dentaire selon l'une quelconque des revendications précédentes, dans lequel le composé dendritique correspond essentiellement à la formule (I)

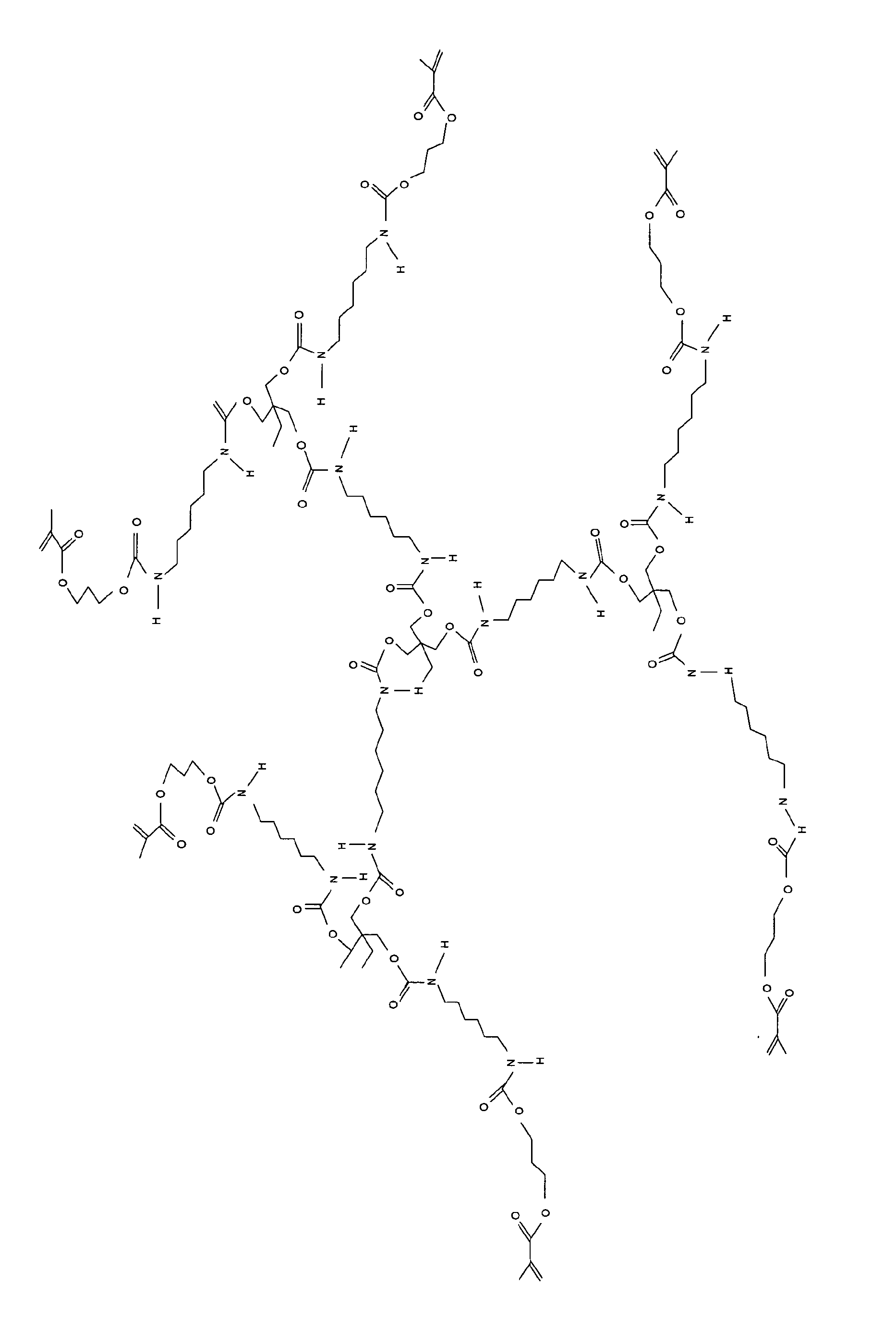

5. Utilisation d'un composé dendritique comme décrit dans l'une quelconque des revendications 1 à 4 précédentes, comme agent d'élasticité dans des matériaux dentaires.

6. Utilisation d'un composé dendritique comme décrit dans l'une quelconque des revendications 1 à 4 précédentes pour améliorer les caractéristiques rhéologiques de matériaux dentaires.
